# EUROPÄISCHE PATENTSCHRIFT

(11) **EP 0 750 490 B1**
(45) Veröffentlichungstag und Bekanntmachung des Hinweises auf die Patenterteilung: **08.11.2000**
(21) Anmeldenummer: 95911309.3
(22) Anmeldetag: 08.03.1995
(51) Int. Cl.: A61K 7/13

(54) **ISATINDERIVATE ZUM FÄRBEN VON KERATINHALTIGEN FASERN**
ISATIN DERIVATIVES FOR DYEING KERATIN-CONTAINING FIBRES
DERIVES D'ISATINE UTILISES POUR TEINDRE DES FIBRES KERATINIQUES

(30) Priorität: 17.03.1994 DE 4409143
(43) Veröffentlichungstag der Anmeldung: 02.01.1997
(73) Patentinhaber: Henkel Kommanditgesellschaft auf Aktien, 40191 Düsseldorf (DE)
(72) Erfinder: MÖLLER, Hinrich, D-40789 Monheim (DE); HÖFFKES, Horst, D-40595 Düsseldorf (DE)
(86) Internationale Anmeldenummer: EP9500848
(87) Internationale Veröffentlichungsnummer: WO9524886

(56) Entgegenhaltungen:
- WO-A-93/19725

## Beschreibung

Gegenstand der Erfindung ist die Verwendung von bestimmten N-substituierten Isatinderivaten zum Färben von keratinhaltigen Fasen sowie diese Isatinderivate enthaltende Färbemittel.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Mit Oxidationsfarbstoffen lassen sich zwar intensive Färbungen mit guten Echtheitseigenschaften erzielen, die Entwicklung der Farbe geschieht jedoch unter dem Einfluß von Oxidationsmitteln wie z. B. H₂O₂, was häufig Schädigungen der Faser zur Folge hat. Direktziehende Farbstoffe werden unter schonenderen Bedingungen appliziert, ihr Nachteil liegt jedoch darin, daß die Färbungen häufig nur über unzureichende Echtheitseigenschaften verfügen.

Färbesysteme auf Basis von Isatin oder Isatinderivaten bieten hier eine Alternative. Isatin ist als Direktfarbstoff zum Farben von Keratinfasern alleine oder in Kombination mit Chinonfarbstoffen in der deutschen Offenlegungsschrift DE 36 35 147 A1 beschrieben worden. Die Variationsbreite der erzielbaren Nuancen ist jedoch beschränkt. In den allermeisten Fällen erhält man eine goldfarbene Färbung.

Ein weiteres isatinhaltiges Färbesystem wird in der europäischen Offenlegungsschrift EP 359 465 A2 beschrieben. Hier wird die Färbung mit Hilfe eines aus der Reaktion eines Isatins mit einem Anilinderivat entstehenden Ketimins (Schiffsche Base) erzielt. Das Ketimin wird entweder als solches auf keratinische Fasern aufgebracht und entwickelt dort eine Färbung, oder aber eine aus einem Isatin und einem Anilinderivat bestehende Mischung wird auf die Faser aufgebracht und bildet zunächst "In situ" das Ketimin, woraufhin sich auf der Faser die Färbung entwickelt.

Die europäische Offenlegungsschrift EP 497 697 A1 beschreibt Haarfärbemittel auf Basis von Isatinen und Aminoindolen oder -indolinen mit primärer Aminogruppe, wobei sich in einer Kondensationsreaktion Schiffsche Basen bilden.

Die europäische Offenlegungsschrift EP 0 502 783 A1 beschreibt Haarfärbemittel, die Isatine und Aminopyridine oder Isatine und Aminopyrimidine mit primärer Aminogruppe enthalten.

Die europäische Offenlegungsschrift EP 0 502 784 A1 beschreibt Haarfärbemittel, die Isatine und substituierte Diamine oder Aminophenole oder aber Isatine und (Bisaryl)-alkylendiamine enthalten.

Die mit diesen Isatinen erzielbaren Haarausfärbungen sind jedoch im Hinblick auf die Brillanz der Farben und Farbintensitäten nicht immer zufriedenstellend.

Aufgabe der vorliegenden Erfindung ist es, Haarfärbesysteme auf Basis von Isatinen zu finden, die besonders farbintensive Ausfärbungen ergeben. Überraschenderweise wurde nun gefunden, daß bestimmte N-substituierte Isatinderivate diesen Anforderungen in hervorragender Weise genügen und sich hervorragend zum Färben von Keratinfasern eignen.

Als keratinhaltige Fasern kommen z.B. Wolle, Pelze, Felle und menschliche Haare in Betracht. Die unten näher bezeichneten N-substituierten Isatinderivate können prinzipiell aber auch zum Färben anderer Naturfasern, wie z.B. Baumwolle, Jute, Sisal, Leinen oder Seide, modifizierter Naturfasern, wie z.B. Regeneratcellulose, Nitro-, Alkyl- oder Hydroxyalkyl- oder Acetylcellulose und synthetischer Fasern, wie z.B. Polyamid-, Polyacrylnitril,-, Polyurethan- und Polyesterfasern verwendet werden.

Gegenstand der Erfindung ist die Verwendung von Isatinderivaten der Formel I 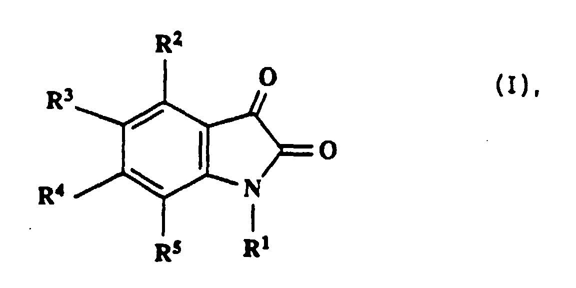 wobei R¹ für eine Hydroxy-, eine gegebenenfalls mit C₁-C₄-Alkyl oder Phenyl substituierte Aminogruppe, eine C₃-C₈-Alkenyl-, Dihydroxy-(C₃-C₆)-alkyl-, Trihydroxy-(C₄-C₆)-alkyl-, Tetrahydroxy-(C₅-C₆)-alkyl-, Pentahydroxy-C₆-alkyl-, C₂-C₄-Aminoalkyl-, C₁-C₄-Sulfoalkylgruppe, einen gegebenenfalls mit C₁-C₄-Alkyl substituierten 2-Furylmethyl-, 2-Thienylmethyl-, 2-Pyridylmethyl-, 3-Pyridylmethyl-, 4-Pyridylmethylrest oder eine Aralkylgruppe der Formel (II) steht 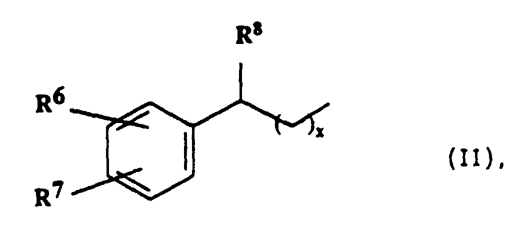 in der R⁶ und R⁷ unabhängig voneinander für Wasserstoffe, Halogenatome, Hydroxygruppen, Aminogruppen, die gegebenenfalls mit C₁-C₄-Alkyl oder Phenyl substituiert sein können, C₁-C₄-Alkylgruppen, C₁-C₄-Alkoxygruppen, Carboxygruppen oder Sulfogruppen stehen und R⁸ für Wasserstoff, eine C₁-C₄-Alkylgruppe, oder eine C₂-C₄-Hydroxyalkylgruppe stehen und x 0, 1 oder 2 bedeutet,
und R², R³, R⁴ und R⁵ unabhängig voneinander Wasserstoffe, Hydroxygruppen, Halogenatome, Nitrogruppen, Sulfogruppen, Carboxylgruppen, C₁-C₄-Alkylgruppen, C₁-C₄-Alkoxygruppen oder NR⁹R¹⁰-Gruppen bedeuten wobei R⁹ und R¹⁰ unabhängig voneinander Wasserstoff, C₁-C₄-Alkylgruppen oder C₂-C₄-Hydroxyalkylgruppen darstellen, und zwei benachbarte Gruppen R³, R⁴ und R⁵ auch eine Alkylendioxygruppe mit 1 bis 4 C-Atomen darstellen können, und deren wasserlöslichen Salzen zum Färben von keratinhaltigen Fasern.

Als wasserlösliche Salze sind ohne Einschränkung z.B. die Alkali- oder Ammoniumsalze von sauren Isatinen der Formel I bzw. die Hydrochloride, Hydrobromide oder Hydrogensulfate von basischen Isatinen der Formel I zu nennen.

Die Isatine der Formel I sind entweder literaturbekannte Verbindungen oder lassen sich durch literaturbekannte Standardsynthesen darstellen, u.a. durch Reaktion von Isatin bzw. im Benzolkern substituierten Isatin-Derivaten oder ihren Natriumsalzen mit entsprechenden Halogen- oder Epoxyverbindungen. Beispiele für Isatine der Formel I sind N-(2,3-Dihydroxypropyl)-, N-(2-Sulfoethyl)-, (3-Sulfopropyl)-, N-Allyl-, N-(2-Dimethylamino)-, N-(2-Pyrrolidino)-, N-(2-Piperidino)-, (2-Morpholinoethyl)-, N-(2-Furylmethyl)-, N-(2-Thienylmethyl)-, N-(2-, N-(3- oder N-(4-Pyridylmethyl)-isatin, N-Allylisatin-5-sulfonsäure, 5-Chlor-, 5-Methyl-N-(hydroxyethyl)-isatin, 5,7-Dichlor-, 5-Nitro-N-allylisatin und die Alkalisalze der sauren Verbindungen.

Vorzugsweise werden zum Färben von keratinhaltigen Fasern diejenigen Isatinderivate der Formel I verwendet, in denen R¹ für eine Allylgruppe, eine Hydroxygruppe oder eine 2-Sulfoethyl- oder -propylgruppe steht und R² bis R⁵ Wasserstoffe bedeuten.

Die Isatinderivate der Formel I ergeben Nuancen im Gelb-Bereich. Besonders brillante Färbungen im Gelb-, Rot-, Violett- und Schwarzbereich mit guten Echtheitseigenschaften (Lichtechtbeit, Waschechtheit, Reibechtheit) werden erzielt, wenn die Isatinderivate der Formel I gemeinsam mit aminogruppenhaltigen Verbindungen, z.B. primären aliphatischen Aminen, die in der C-Kette mindestens eine zusätzliche Amino- oder C₁-C₄-Alkoxygruppe tragen, Di- und Trihydroxyaromaten, z.B. Pyrogallol, Hydroxyhydrochinon, Phloroglucin, Resorcin, 2-, 4-Methylresorsin, 3-Dimethylaminophenol oder gemeinsam mit heterocyclischen aromatischen Verbindungen verwendet werden.

Besonders geeignete amonogruppenhaltige Verbindungen sind Aminosäuren und Oligopeptide. Ein weiterer Erfindungsgegenstand sind deshalb Mittel zum Färben von keratinhaltigen Fasern, enthaltend mindestens ein Isatinderivat der Formel I und mindestens eine Aminosäure oder ein aus 2 bis 9 Aminosäuren aufgebautes Oligopeptid.

Als Aminosäuren kommen alle natürlich vorkommenden und synthetischen Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein zugänglichen Aminosäuren. Dabei können sowohl sauer als auch alkalisch reagierende Aminosäuren eingesetzt werden.

Geeignete Oligopeptide sind alle aus natürlich vorkommenden und synthetischen Aminosäuren aufgebauten Oligopeptide. Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mandelprotein enthaltenen Oligopeptide zu nennen.

Zum Einsatz in den erfindungsgemäßen Färbemitteln eignen sich jedoch besonders diejenigen Aminosäuren oder Oligopeptide, die ausgewählt sind aus der Gruppe Arginin, Cystein, Methionin, Prolin, Tyrosin, Valin, Glycin, Glutaminsäure, Histidin, Asparaginsäure, Alanin, Tryptophan, Cystin, Lysin, Hydroxyprolin, Leucin, Isoleucin, Phenylalanin, 3,4-Dihydroxyphenylalanin, Serin, Ornithin, Threonin, Glutathion.

Ein weiterer Erfindungsgegenstand sind Mittel zum Färben von keratinhaltigen Fasern enthaltend mindestens ein Isatinderivat der Formel I und mindestens ein aromatisches Amin der Formel III 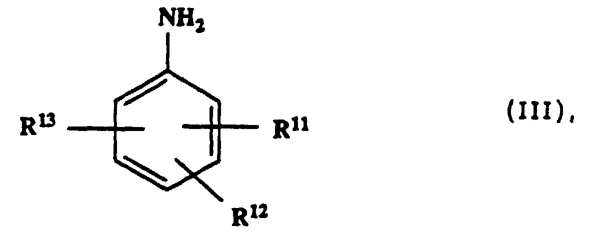 wobei R¹¹, R¹² und R¹³ Wasserstoffe, C₁-C₄-Alkylgruppen, C₁-C₄-Alkoxygruppen, Hydroxygruppen, C₂-C₄-Hydroxyalkylgruppen, Carboxylgruppen, Sulfogruppen, C₁-C₄-Aminoalkylgruppen oder NR¹⁴R¹⁵-Gruppen darstellen, wobei R¹⁴ und R¹⁵ unabhängig voneinander für Wasserstoffe, C₁-C₄-Alkylgruppen, Arylgruppen, oder C₂-C₄-Hydroxyalkylgruppen stehen,
wobei zwei der Gruppen R¹¹, R¹² und R¹³ gemeinsam auch einen ankondensierten Benzolring bilden können, der gegebenenfalls mit C₁-C₄-Alkyl, Hydroxy, Carboxyl, Sulfo, C₁-C₄-Aminoalkyl oder Amino substituiert ist.

Beispiele sind z. B. p-Phenylendiamin, 3-Amino-6-methylphenol, o-Phenylendiamin, Sulfanilsäure, 1,5-, 1,8-, 2,3-Diaminonaphthalin, 6-Amino-1-naphthol-3-sulfonsäure, 4-Amino-, 4,4'-Diaminodiphenylamin, 4,4'-Diaminodiphenylamin-2-sulfonsäure, 4-Aminodiphenylamin, N,N'-Bis-(2-hydroxyethyl)-p-phenylendiamin, 4-Dimethylamino-anilin.

Bevorzugt sind diejenigen Färbemittel, in denen das aromatische Amin der Formel III ausgewählt ist aus der Gruppe p-Toluylendiamin, 4-Aminophenol, 4-Amino-2-aminomethylphenol, 3,4-Diaminobenzoesäure, 1-(β-Hydroxyethyl)-2,5-diaminobezol.

Ein weiterer Erfindungsgegenstand sind Mittel zum Färben von keratinhaltigen Fasern enthaltend mindestens ein Isatinderivat der Formel I und mindestens ein aromatisches Amin der Formel IV 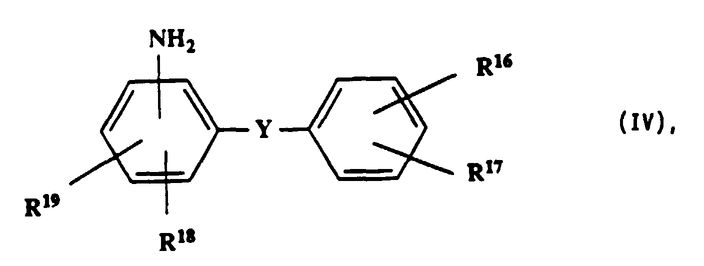 wobei Y für eine direkte Bindung oder für eine Gruppe CO, SO, O, S, NR²⁰ mit R²⁰ gleich Wasserstoff, C₁-C₄-Alkyl oder C₂-C₄-Hydroxyalkyl oder O-(CH₂-Z-CH₂-O)ₘ steht, wobei Z für eine direkte Bindung, eine Gruppe CH₂, CHOH oder CH₂OC₂H₄OCH₂ steht, und m eine ganze Zahl von 1 bis 4 bedeutet oder Y auch eine gesättigte oder ungesättigte Alkylengruppe mit 1 bis 4 C-Atomen, die gegebenenfalls durch OH substituiert sein kann, bedeutet und R¹⁶, R¹⁷, R¹⁸ und R¹⁹ Wasserstoffe, C₁-C₄-Alkylgruppen, C₂-C₄-Hydroxyalkylgruppen, C₂-C₄-(C₁-C₄-Alkoxy)-alkylgruppen oder Gruppen NR²¹R²² oder OR²³ darstellen, wobei R²¹, R²² und R²³ unabhängig voneinander Wasserstoffe, C₁-C₄-Alkylgruppen, C₁-C₄-Alkoxygruppen, C₂-C₄-Hydroxyalkylgruppen oder C₂-C₄-(C₁-C₄-Alkoxy)-alkylgruppen bedeuten, mit der Maßgabe, daß jeweils mindestens eine der Gruppen R¹⁶ und R¹⁷ und eine der Gruppen R¹⁸ und R¹⁹ eine Gruppe NR²¹R²² oder OR²³ darstellt.

Bevorzugt sind diejenigen Färbemittel, in denen das aromatische Amin der Formel III 1,3-Bis-(2,4-diaminophenoxy)propan, 1,8-Bis-(2,5-di-aminophenoxy)-3,6-dioxaoctan oder 4,4'-Diaminodiphenylamin ist.

Ein weiterer Erfindungsgegenstand sind Mittel zum Färben von keratinhaltigen Fasern enthaltend mindestens ein Isatinderivat der Formel I und mindestens ein Aminopyrimidin der Formel V 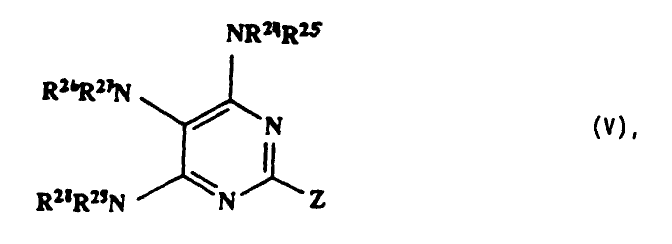 wobei R²⁴, R²⁵, R²⁶, R²⁷, R²⁸ und R²⁹ unabhängig voneinander Wasserstoffe, C₁-C₄-Alkylgruppen oder C₂-C₄-Hydroxyalkylgruppen darstellen und Z für Wasserstoff, eine OH- oder eine NR³⁰R³¹-Gruppe steht, in der R³⁰ und R³¹ unabhängig voneinander Wasserstoffe, C₁-C₄-Alkylgruppen oder C₂-C₄-Hydroxyalkylgruppen darstellen.

Bevorzugt sind dabei diejenigen Färbemittel, in denen unsubstituiertes 2,4,5,6-Tetraaminopyrimidin eingesetzt wird.

Weitere gemeinsam mit Isatinderivaten der Formel I zu verwendende Substanzen sind Heterocyclen z.B. Pyrrol, 25-Dimethyl-3-ethyl-pyrrol, 2-, 3-, 4-Amino-, 2-Amino-3-hydroxy-, 2,6-Diamino-, 2,5-Diamino-, 2,3-Diamino-, 2-Dimethylamino-5-amino-, 3-Aznino-2-methyl-amino-6-methoxy-, 2,3-Diamino-6-methoxy-, 2,4,5-Triamino-, 2,6-Dihydroxy-3,4-dimethylpyridin, 4,5,6-Triamino-, 4-Hydroxy-2,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 3,5-Diamino-pyrazol, -1,2,4-triazol, 3-Amino-, 3-Amino-5-hydroxypyrazol, 2-, 3-, 8-Aminochinolin, 4-Amino-chinaldin, 2-, 6-Aminonicotinsäure, 5-Aminoisochinolin, 4-, 5-, 6-, 7-Aminoindol, 5-, 6-Aminoindazol, 5-, 7-Amino-benzimidazol, -benzothiazol. Besonders eignen sich jedoch Indol- und Indolinderivate.

Ein weiterer Erfindungsgegenstand sind Mittel zum Färben von keratinhaltigen Fasern enthaltend mindestens ein Isatinderivat der Formel I und
- mindestens ein Indolderivat der Formel VIa 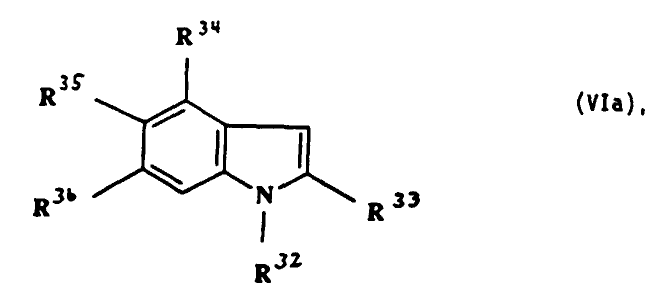 wobei R³² Wasserstoff, eine C₁-C₄-Alkyl- oder C₂-C₄-Acylgruppe, R³³ Wasserstoff oder eine Carboxylgruppe bedeutet und R³⁴, R³⁵ und R³⁶ Wasserstoffe, C₁-C₄-Alkylgruppen, C₂-C₄-Acyloxygruppen, Hydroxygruppen, Aminogruppen oder C₁-C₄-Alkoxygruppen darstellen, oder mindestens ein Indolinderivat der Formel VIb 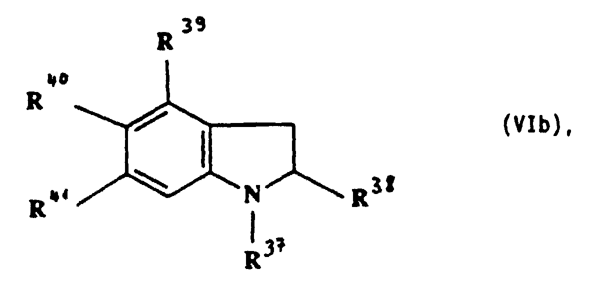 wobei R³⁷ Wasserstoff , eine C₁-C₄-Alkyl- oder C₂-C₄-Acylgruppe, R³⁸ Wasserstoff oder eine Carboxylgruppe bedeutet und R³⁹, R⁴⁰ und R⁴¹ Wasserstoffe, C₁-C₄-Alkylgruppen, C₂-C₄-Acyloxygruppen, Hydroxygruppen, Aminogruppen oder C₁-C₄-Alkoxygruppen darstellen.

Bevorzugt sind diejenigen Färbemittel, in denen das Indolderivat der Formel Va bzw. das Indolinderivat der Formel Vb ausgewählt ist aus der Gruppe 5,6-Dihydroxyindol, 5,6-Diacetoxyindol, 4-Hydroxyindolin, 6-Aminoindolin und 5,6-Dihydroxyindolin.

In allen erfindungsgemäßen Färbemitteln können mehrere verschiedene Isatinderivate der Formel I gemeinsam zum Einsatz kommen; ebenso können auch mehrere verschiedene Aminosäuren bzw. Oligopeptide sowie mehrere verschiedene Verbindungen der Formeln III, IV, V, VIa und VIb gemeinsam verwendet werden.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren.

Zur Anwendung auf dem menschlichen Haar können die erfindungsgemäßen Färbemittel in einen wasserhaltigen kosmetischen Träger eingearbeitet werden. Geeignete wasserhaltige kosmetische Träger sind z.B. Cremes, Emulsionen, Gele oder auch tensidhaltige schäumende Lösungen wie z.B. Shampoos oder andere Zubereitungen, die für die Anwendung auf dem Haar geeignet sind. Die Isatine der Formel I sowie die Aminosäuren und Oligopeptide bzw. die Verbindungen der Formeln III, IV, V, VIa und VIb sind dabei in einer Menge von jeweils 0,1 bis 20, vorzugsweise 1 bis 7 Gew.-%, jeweils bezogen auf die gesamte Färbemittelzubereitung, enthalten.

Der wasserhaltige kosmetische Träger enthält üblicherweise Netz- und Emulgiermittel wie anionische, nichtionische oder ampholytische Tenside, z.B. Fettalkoholsulfate, Alkansulfonate, α-Olefinsulfonate, Fettalkoholpolyglykolethersulfate, Alkylglycoside, Ethylenoxidanlagerungsprodukte an Fettalkohole, an Fettsäuren, an Alkylphenole, an Sorbitanfettsäureester, an Fettsäurepartialglyceride und Fettsäurealkanolamide; Verdickungsmittel, z. B. Fettalkohole, Fettsäuren, Paraffinöle, Fettsäureester und andere Fettkomponenten in emulgierter Form; wasserlösliche polymere Verdickungsmittel wie natürliche Gummen, z. B. Gummi arabicum, Karaya-Gummi, Guar-Gummi, Johannisbrotkernmehl, Leinsamengummen und Pektin, biosynthetische Gummen, z.B. Xanthan-Gummi und Dextrane, synthetische Gummen, z. B. Agar-Agar und Algin, Stärke-Fraktionen und Derivate wie Amylose, Amylopektin und Dextrine, modifizierte Cellulosemoleküle, z. B. Methylcellulose, Hydroxyalkylcellulose und Carboxymethylcellulose, Tone wie z. B. Bentonit oder vollsynthetische Hydrokolloide, z.B. Polyvinylalkohol oder Polyvinylpyrrolidon, haarpflegende Zusätze, wie z.B. wasserlösliche kationische Polymere, anionische Polymere, nichtionische Polymere, amphotere oder zwitterionische Polymere, Pantothensäure, Vitamine, Pflanzenextrakte oder Cholesterin, pH-Stellmittel, Komplexbildner und Parfumöle sowie Reduktionsmittel zur Stabilisierung der Inhaltsstoffe, z. B. Ascorbinsäure, schließlich können auch Farbstoffe zum Einfärben der kosmetischen Zubereitungen enthalten sein.

Der pH-Wert der gebrauchsfertigen Färbezubereitungen liegt zwischen 2 und 11, vorzugsweise zwischen 5 und 9. Bei Verwendung von luftoxidablen Komponenten wie z.B. 5,6-Dihydroxyindol, 5,6-Dihydroxyindolin und deren Derivaten ist ein schwach alkalischer pH-Wert von 7 - 11, vorzugsweise 8 - 10, vorteilhaft.

Zum Haarefärben werden die erfindungsgemäßen Färbemittel in Form des wasserhaltigen, kosmetischen Trägers in einer Menge von 100 g auf das Haar aufgebracht, ca. 30 Minuten dort belassen und dann ausgespült oder mit einem handelsüblichen Haarsphampoo ausgewaschen.

Wenn die erfindungsgemäßen Färbemittel in Form einer wasserhaltigen kosmetischen Zubereitung vorliegen, ist es vorteilhaft (aber nicht in jedem Falle notwendig), das Isatinderivat der Formel I getrennt von der zweiten reaktiven Komponente, d.h. der Aminosäure oder dem Oligopeptid bzw. der Verbindungen der Formeln III, IV, V, VIa und VIb, zu konfektionieren.

Die beiden Komponenten (Isatinderivat der Formel I und zweite reaktive Komponente) können dann entweder gleichzeitig auf das Haar aufgebracht werden oder aber auch nacheinander, wobei es keine Rolle spielt, welche der beiden Komponenten zuerst aufgetragen wird; zwischen dem Auftragen der ersten und der zweiten Komponente können bis zu 30 Minuten Zeitabstand liegen.

Besonders vorteilhaft ist es jedoch, alle Komponenten gemeinsam in einer wasserfreien, pulverförmigen Zubereitung zu konfektionieren.

Ein weiterer Erfindungsgegenstand sind deshalb Haarfärbemittel in Form eines Pulvers enthaltend mindestens ein Isatinderivat der Formel I in einer Menge von 1 bis 90 Gew.-%, vorzugsweise 20 bis 50 Gew.-%, und eine Aminosäure oder ein aus 2 bis 9 Aminosäuren aufgebautes Oligopeptid in einer Menge von 1 bis 90 Gew.-%, vorzugsweise 20 bis 50 Gew.-%, jeweils bezogen auf das gesamte Pulverfärbemittel.

Besonders bevorzugt sind dabei solche Haarfärbemittel, in denen das Isatinderivat der Formel I ausgewählt ist aus N-Allylisatin, N-Hydroxyisatin und N-(2-Sulfoethyl)-isatin und die Aminosäure bzw. das Oligopeptid ausgewählt sind aus Arginin, Cystein, Methionin, Prolin, Tyrosin, Valin, Glycin, Glutaminsäure, Histidin, Asparaginsäure, Alanin, Tryptophan, Cystin, Lysin, Hydroxyprolin, Leucin, Isoleucin, Phenylalanin, 3,4-Dihydroxyphenylalanin, Serin, Ornithin, Threonin, Glutathion.

Ein weiterer Erfindungsgegenstand sind Haarfärbemittel in Form eines Pulvers enthaltend mindestens ein Isatinderivat der Formel I in einer Menge von 1 bis 90 Gew.-%, vorzugsweise 20 bis 50 Gew.-%, und mindestens ein aromatisches Amin der Formel III in einer Menge von 1 bis 90 Gew.-%, vorzugsweise 20 bis 50 Gew.-%, jeweils bezogen auf das gesamte Pulverfärbemittel.

Besonders bevorzugt sind dabei solche Haarfärbemittel, in denen das Isatinderivat der Formel I ausgewählt ist aus N-Allylisatin, N-Hydroxyisatin, N-(2-Sulfoethyl)-isatin und N-(2-Sulfopropyl)-isatin und das aromatische Amin der Formel III ausgewählt ist aus der Gruppe p-Toluylendiamin, 4-Aminophenol, 4-Amino-2-aminomethylphenol, 3,4-Diaminobenzoesäure, 1-(β-Hydroxyethyl)-2,5-diaminobenzol.

Ein weiterer Erfindungsgegenstand sind Haarfärbemittel in Form eines Pulvers enthaltend mindestens ein Isatinderivat der Formel I in einer Menge von 1 bis 90 Gew.-%, vorzugsweise 20 bis 50 Gew.-%, und mindestens ein aromatisches Amin der Formel IV in einer Menge von 1 bis 90 Gew.-%, vorzugsweise 20 bis 50 Gew.-%, jeweils bezogen auf das gesamte Pulverfärbemittel.

Besonders bevorzugt sind dabei solche Haarfärbemittel, in denen das Isatinderivat der Formel I ausgewählt ist aus N-Allylisatin, N-Hydroxyisatin, N-(2-Sulfoethyl)-isatin und N-(2-Sulfopropyl)-isatin und das aromatische Amin der Formel IV 1,3-Bis-(2,4-diaminophenoxy)-propan ist.

Ein weiterer Erfindungsgegenstand sind Haarfärbemittel in Form eines Pulvers enthaltend mindestens ein Isatinderivat der Formel I in einer Menge von 1 bis 90, vorzugsweise 20 bis 50 Gew.-%, und mindestens ein Aminopyrimidin der Formel V in einer Menge von 1 bis 90 Gew.-%, vorzugsweise 20 bis 50 Gew.-%, jeweils bezogen auf das gesamte Pulverfärbemittel.

Besonders bevorzugt sind dabei solche Haarfärbemittel, in denen das Isatinderivat der Formel I ausgewählt ist aus N-Allylisatin, N-Hydroxyisatin, N-(2-Sulfoethyl)-isatin und N-(2-Sulfopropyl)-isatin und das Indolderivat der Formel VIa bzw. das Indolinderivat der Formel VIb ausgewählt sind aus 5,6-Dihydroxyindol, 5,6-Diacetoxyindol, 4-Hydroxyindolin, 6-Aminoindolin und 5,6-Dihydroxyindolin.

Die Isatine der Formel I können vorteilhafterweise auch mit Pyridinen wie z. B. 2,6-Dihydroxy-3,4-dimethyl-, 2-Amino-3-hydroxy-, 2-Methylamino-3-amino-6-methoxy-, 3,5-Diamino-2,6-dimethoxypyridin als zweiter Komponente kombiniert werden.

Im einfachsten Falle enthalten die pulverförmigen Haarfärbemittel außer den beiden reaktiven Komponenten (Isatinderivat der Formel I und Aminosäure oder Oligopeptid bzw. Verbindungen der Formeln III, IV, V, VIa und VIb) lediglich ein wasserlösliches polymeres Verdickungsmittel. Das Verdickungsmittel hat die Aufgabe, der nach Zugabe von Wasser erhaltenen gebrauchsfertigen Haarfärbemittelzubereitung die anwendungstechnisch gewünschte Konsistenz zu verleihen. Geeignete Verdickungsmittel sind z.B. natürliche Gummen, z. B. Guarkernmehl, Gummi arabicum, Karaya-Gummi, Johannisbrotkernmehl, Leinsamengummen und Pektin, biosynthetische Gummen wie z.B. Xanthan-Gummi und Dextrane, synthetische Gummen wie z.B. Agar-Agar und Algin, Stärke-Fraktionen und Derivate wie Amylose, Antylopektin und Dextrine, modifizierte Cellulosemoleküle, z. B. Methylcellulose, Hydroxyalkylcellulose und Carboxymethylcellulose.

Weitere fakultative Komponenten in dem Pulverhaarfärbemittel sind Metallsalze, z.B. die Acetate, Sulfate, Chloride, Bromide, Carbonate, Glykolate, Lactate oder Gluconate der Alkali-, Erdalkalimetalle sowie von Zink und Mangan-(II), weiterhin Tenside wie z.B. anionische, nichtionogene, amphotere oder zwitterionische bzw. kationische Tenside, haarpflegende Zusätze wie z.B. wasserlösliche kationische Polymere, anionische Polymere, amphotere oder zwitterionische Polymere, Pantothensäure, Vitamine, Pflanzenextrakte, Komplexbildner, Parfümkomponenten sowie Reduktionsmittel wie z.B. Ascorbinsäure.

Zur Bereitung der gebrauchsfertigen Haarfärbemittel-Zubereitung werden 1 bis 30 g, vorzugsweise 3 bis 15 g des Pulvers mit heißem Wasser von 90 bis 100°C auf 100 g aufgefüllt.Zum Lösen des Pulvers können auch Wasser/Alkohol-Gemische oder andere kosmetisch verträgliche Lösungsmittel verwendet werden. Einzelne Bestandteile der Mischung können dabei auch getrennt trocken vermengt und gelagert werden.

Nach Abkühlen auf ca. 40°C wird die gebrauchsfertige Haarfärbemittel-Zubereitung auf das Haar aufgebracht und dort 30 Minuten lang belassen. Anschließend wird das Haar gespült oder mit einem handelsüblichen Haarshampoo gewaschen.

Als eine besondere Form der Ausfärbung ist es auch möglich, die Komponenten in wäßrigem Medium zum Sieden zu erhitzen und miteinander reagieren zu lassen, das Reaktionsgemisch abzukühlen, den Niederschlag abzufiltrieren und diesen in wäßriger Dispersion als direktziehenden Farbstoff zum Färben der Haare einzusetzen.

Die folgenden Beispiele sollen den Erfindungsgegenstand näher erläutern, ohne ihn jedoch hierauf zu beschränken.

### Beispiele

Es wurde eine Aufschlämmung von 10 mMol eines Isatins der Formel I und 10 mMol einer zweiten Komponente in 100 ml Wasser bereitet. Die Aufschlämmung wurde auf Siedetemperatur erhitzt und nach dem Abkühlen filtriert, der pH-Wert wurde anschließend auf 6 eingestellt. In diese Färbelösung wurden bei 30°C 30 Minuten lang zu 90 % ergraute, nicht vorbehandelte Menschenhaare eingebracht. Die jeweiligen Färbetemperaturen, Färbedauern, Farbnuancen und Farbtiefen sind den Tabellen 1 - 3 zu entnehmen.

Die Farbtiefe wurde dabei nach folgender Skala bewertet:

| | |
|---|---|
| - | keine oder eine sehr blasse Ausfärbung |
| (+) | schwache Intensität |
| + | mittlere Intensität |
| +(+) | mittlere bis starke Intensität |
| ++ | starke Intensität |
| ++(+) | starke bis sehr starke Intensität |
| +++ | sehr starke Intensität |

**Tabelle 1**

| **Ausfärbungen mit N-Allylisatin** | | |
|---|---|---|
| Zweite Komponente | Färbenuance | Farbtiefe |
| - | blaßgelb | (+) |
| 2,5-Diaminotoluol H₂SO₄ | rotbraun | ++(+) |
| 2,4,5,6-Tetraaminopyrimidin H₂SO₄ | dunkelorange | ++(+) |
| 3,4-Diaminobenzoesäure | hellbraunorange | + |
| 2-(2,5-Diaminophenyl)-ethanol H₂SO₄ | braunrot | ++(+) |
| 1,8-Bis-(2,5-diaminophenoxy)-3,6-dioxaoctan 4HCl | dunkelgrau | ++(+) |
| 2-Aminoimethyl-3-amino-6-methoxypyrimidin 2HCl | dunkelgelbgrau | ++(+) |
| 2-Aminomethyl-4-aminophenol 2HCl | orange | ++ |
| 1,3-Bis-(2,4-diaminophenoxy)-propan 4HCl | beigegrau | + |
| 4-(4-Amino-m-toluidino)-phenol | hellgrauviolett | (+) |
| N,N-Dimethyl-p-phenylendiamin H₂SO₄ | dunkelviolett | +++ |
| N-Phenyl-p-phenylendiamin HCl | hellbraun | +(+) |
| 4,4'-Diaminodiphenylamin H₂SO₄ | dunkelbiolettblau | +++ |
| N-(4-Methoxyphenyl)-p-phenylendiamin HCl | hellbraun | +(+) |
| 4,4'-Diaminodiphenylamin-2-sulfonsäure | violettrot | +++ |
| 2,4-Dimethyl-3-ethylpyrrol | gelb | + |
| 1-(o-Aminophenyl)-pyrrol | gelb | +(+) |
| N-Methylpyrrol | gelb | +(+) |
| L-Histidin | olivgelb | + |
| L-Arginin | rosa | + |
| L-Tryptophan | blaßgelb | (+) |
| Pyrrol | olivgelb | (+) |
| L-Ornithin HCl | blaßgelb | (+) |
| 2,6-Dimethoxy-3,5-diaminopyridin HCl | grünschwarz | +++ |

**Tabelle 2**

| **Ausfärbungen mit N-Hydroxyisatin** | | |
|---|---|---|
| Zweite Komponente | Färbenuance | Farbtiefe |
| - | hellgelb | (+) |
| p-Toluylendiamin H₂SO₄ | kupfer | ++ |
| Tetraaminopyrimidin H₂SO₄ | orangekupfer | ++ |
| 2-(2,5-Diaminophenyl)-ethanol H₂SO₄ | kupfer | ++ |
| 1,8-Bis-(2,5-Diaminophenoxy-3,6-dioxaoctan 4HCl | violettbraun | ++ |

**Tabelle 3**

| **Ausfärbungen mit N-(2-Sulfoethyl)-isatin** | | |
|---|---|---|
| Zweite Komponente | Farbnuance | Farbtiefe |
| - | gelb | + |
| 2,5-Diaminotoluol H₂SO₄ | violettrot | ++(+) |
| 2,4,5,6-Tetraaminopyrimidin H₂SO₄ | dunkelkupfer | ++ |
| 3,4-Diaminobenzoesäure | gelbbeige | (+) |
| 2-(2,5-Diaminophenyl)-ethanol H₂SO₄ | kupfer | ++ |
| 1,8-Bis-(2,5-diaminophenoxy)-3,6-dioxaoctan 4HCl | dunkelgrau | ++ |
| 2-Methylamino-3-amino-6-methoxypyridin 2HCl | blaßviolett | (+) |
| 2-Aminomethyl-4-aminophenol 2HCl | tiefgelb | ++ |
| 1,3-Bis-(2,4-diaminophenoxy)-propan 4HCl | olivgrau | + |
| N,N-Dimethyl-p-phenylendiamin H₂SO₄ | rotviolett | ++(+) |
| 4,4-Diaminodiphenylamin H₂SO₄ | dunkelblaugrau | ++(+) |
| 4,4-Diaminodiphenylamin-2-sulfonsäure | hellviolettrot | +(+) |
| 2,4-Dimethyl-3-ethylpyrrol | gelbbraun | ++ |
| L-Tryptophan | braungelb | + |
| L-Tyrosin | gelb | + |
| Pyrrol | blaß | |
| 2,6-Dimethoxy-3,5-diaminopyridin | grünschwarz | +++ |

## Patentansprüche

1. Verwendung von Isatinderivaten der Formel 1 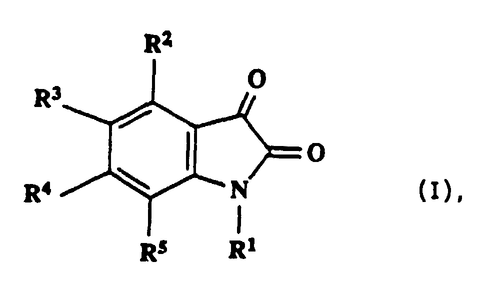 wobei R¹ für eine Hydroxy-, eine gegebenenfalls mit C₁-C₄-Alkyl oder Phenyl substituierte Aminogruppe, eine C₃-C₈-Alkenyl-, Dihydroxy- (C₃-C₆)-alkyl-, Trihydroxy-(C₄-C₆)-alkyl-, Tetrahydroxy-(C₅-C₆)-alkyl-, Pentahydroxy-C₆-alkyl-, C₂-C₄-Aminoalkyl-C₁-C₄-Sulfoalkylgruppe, einen gegebenenfalls mit C₁-C₄-Alkyl substituierten 2-Furylmethyl-, 2-Thienylmethyl-, 2-Pyridylmethyl-, 3-Pyridylmethyl-, 4-Pyridylmethylrest oder eine Aralkylgruppe der Formel (II) steht 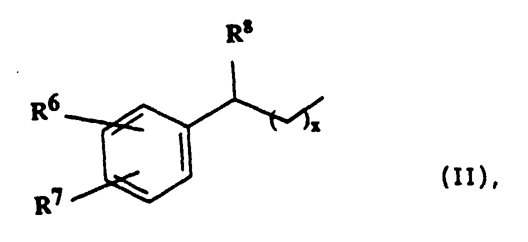 in der R⁶ und R⁷ unabhängig voneinander für Wasserstoffe, Halogenatome, Hydroxygruppen, Aminogruppen, die gegebenenfalls mit C₁-C₄-Alkyl oder Phenyl substituiert sein können, C₁-C₄-Alkylgruppen, C₁-C₄-Alkoxygruppen, Carboxygruppen oder Sulfogruppen stehen und R⁸ für Wasserstoff, eine C₁-C₄-Alkylgruppe, oder eine C₂-C₄-Hydroxyalkylgruppe stehen und x 0,1 oder 2 bedeutet, und R², R³, R⁴ und R⁵ unabhängig voneinander Wasserstoffe, Hydroxygruppen, Halogenatome, Nitrogruppen, Sulfogruppen, Carboxylgruppen, C₁-C₄-Alkylgruppen, C₁-C₄-Alkoxygruppen oder NR⁹R¹⁰-Gruppen bedeuten, wobei R⁹ und R¹⁰ unabhängig voneinander Wasserstoff, C₁-C₄-Alkylgruppen oder C₂-C₄-Hydroxyalkylgruppen darstellen, und zwei benachbarte Gruppen R³, R⁴ und R⁵ auch eine Alkylendioxygruppe mit 1 bis 4 C-Atomen darstellen können, und deren wasserlöslichen Salzen zum Färben von keratinhaltigen Fasern.

2. Verwendung von Isatinderivaten der Formel I nach Anspruch 1, dadurch gekennzeichnet, daß R¹ für eine Allylgruppe, eine Hydroxygruppe oder eine 2-Sulfoethyl- oder -propylgruppe steht und R² bis R⁵ Wasserstoffe darstellen.

3. Mittel zum Färben von keratinhaltigen Fasern, enthaltend mindestens ein Isatinderivat der Formel I und mindestens eine Aminosäure oder ein aus 2 bis 9 Aminosäuren aufgebautes Oligopeptid.

4. Mittel nach Anspruch 3, dadurch gekennzeichnet, daß die Aminosäure oder das Oligopeptid ausgewählt sind aus der Gruppe Arginin, Cystein, Methionin, Prolin, Tyrosin, Valin, Glycin, Glutaminsäure, Histidin, Asparaginsäure, Alanin, Tryptophan, Cystin, Lysin, Hydroxyprolin, Leucin, Isoleucin, Phenylalanin, 3,4-Dihydroxyphenylalanin, Serin, Ornithin, Threonin, Glutathion.

5. Mittel zum Färben von keratinhaltigen Fasern enthaltend mindestens ein Isatinderivat der Formel I und mindestens ein aromatisches Amin der Formel III 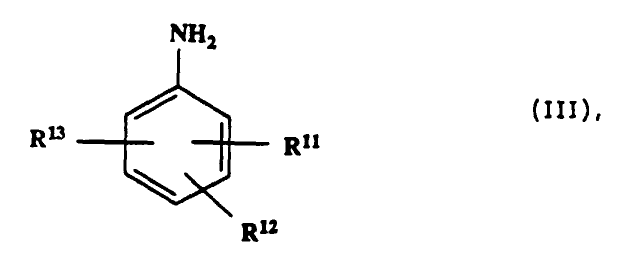 in der R¹¹, R¹² und R¹³ Wasserstoffe, C₁-C₄-Alkylgruppen, C₁-C₄-Alkoxygruppen, Hydroxygruppen, C₂-C₄-Hydroxyalkylgruppen, Carboxylgruppen, Sulfogruppen, C₁-C₄-Aminoalkylgruppen oder NR¹⁴R¹⁵-Gruppen darstellen, wobei R¹⁴ und R¹⁵ unabhängig voneinander für Wasserstoffe, C₁-C₄-Alkylgruppen, Arylgruppen, oder C₂-C₄-Hydroxyalkylgruppen stehen,
wobei zwei der Gruppen R¹¹, R¹² und R¹³ gemeinsam auch einen ankondensierten Benzolring bilden können, der gegebenenfalls mit C₁-C₄-Alkyl, Hydroxy, Carboxyl, Sulfo, C₁-C₄-Aminoalkyl oder Amino substituiert ist.

6. Mittel nach Anspruch 5, dadurch gekennzeichnet, daß das aromatische Amin der Formel III ausgewählt ist aus der Gruppe p-Toluylendiamin, 4-Aminophenol, 4-Amino-2-aminomethylphenol, 3,4-Diaminobenzoesäure, 1-(β-Hydroxyethyl)-2,5-diaminobenzol.

7. Mittel zum Färben von keratinhaltigen Fasern enthaltend mindestens ein Isatinderivat der Formel I und mindestens ein aromatisches Amin der Formel IV 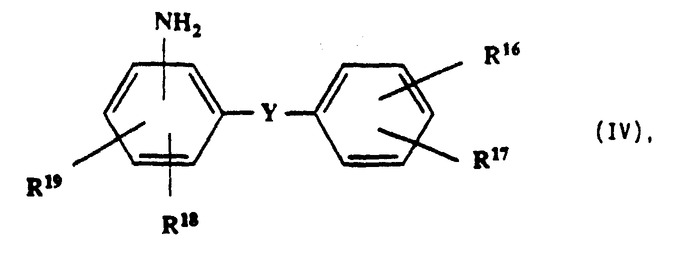 wobei Y für eine direkte Bindung oder für eine Gruppe CO, SO, O, S, NR²⁰ mit R²⁰ gleich Wasserstoff, C₁-C₄-Alkyl oder C₂-C₄-Hydroxyalkyl oder O-(CH₂-Z-CH₂-O)ₘ steht, wobei Z für eine direkte Bindung, eine Gruppe CH₂, CHOH oder CH₂OC₂H₄OCH₂ steht, und m eine ganze Zahl von 1 bis 4 bedeutet oder Y auch eine gesättigte oder ungesättigte Alkylengruppe mit 1 bis 4 C-Atomen, die gegebenenfalls durch OH substituiert sein kann, bedeutet und R¹⁶, R¹⁷, R¹⁸ und R¹⁹ Wasserstoffe, C₁-C₄-Alkylgruppen, C₂-C₄-Hydroxyalkylgruppen, C₂-C₄-(C₁-C₄-Alkoxy)-alkylgruppen oder Gruppen NR²¹R²² oder OR²³ darstellen, wobei R²¹, R²² und R²³ unabhängig voneinander Wasserstoffe, C₁-C₄-Alkylgruppen, C₁-C₄-Alkoxygruppen, C₂-C₄-Hydroxyalkylgruppen oder C₂-C₄-(C₁-C₄-Alkoxy)-alkylgruppen bedeuten, mit der Maßgabe, daß jeweils mindestens eine der Gruppen R¹⁶ und R¹⁷ und eine der Gruppen R¹⁸ und R¹⁹ eine Gruppe NR²¹R²² oder OR²³ darstellt.

8. Mittel nach Anspruch 7, dadurch gekennzeichnet, daß das aromatische Amin der Formel IV 1,3-Bis-(2,4-diaminophenoxy)propan, 1,8-Bis-(2,5-diaminophenoxy)-3,6-dioxaoctan oder 4,4'-Diaminodiphenylamin ist.

9. Mittel zum Färben von keratinhaltigen Fasern enthaltend mindestens ein Isatinderivat der Formel I und mindestens ein Aminopyrimidin der Formel V 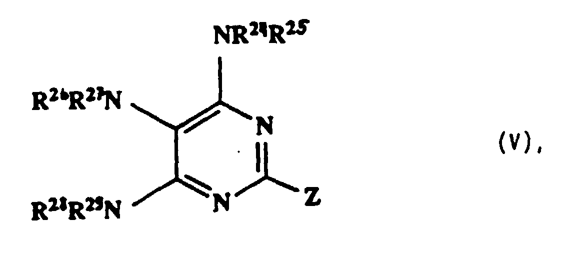 wobei R²⁴, R²⁵, R²⁶, R²⁷, R²⁸ und R²⁹ unabhängig voneinander Wasserstoffe, C₁-C₄-Alkylgruppen oder C₂-C₄-Hydroxyalkylgruppen darstellen und Z für Wasserstoff, eine OH- oder eine NR³⁰R³¹-Gruppe steht, in der R³⁰ und R³¹ unabhängig voneinander Wasserstoffe, C₁-C₄-Alkylgruppen oder C₂-C₄-Hydroxyalkylgruppen darstellen.

10. Mittel nach Anspruch 9, dadurch gekennzeichnet, daß in dem Tetraaminopyrimidin der Formel V die Gruppen R²⁴ bis R²⁹ Wasserstoffe darstellen.

11. Mittel zum Färben von keratinhaltigen Fasern enthaltend mindestens ein Isatinderivat der Formel I und
- mindestens ein Indolderivat der Formel VIa 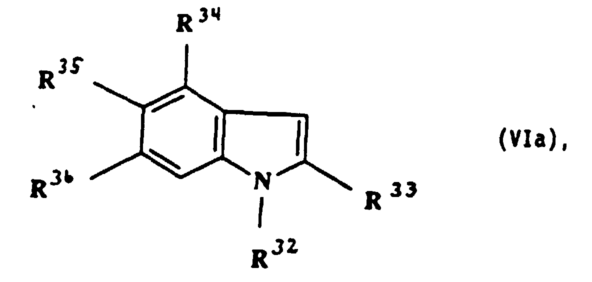 wobei R³² Wasserstoff, eine C₁-C₄-Alkyl- oder C₂-C₄-Acylgruppe, R³³ Wasserstoff oder eine Carboxylgruppe bedeutet und R³⁴, R³⁵ und R³⁶ Wasserstoffe, C₁-C₄-Alkylgruppen, C₂-C₄-Acyloxygruppen, Hydroxygruppen, Aminogruppen oder C₁-C₄-Alkoxygruppen darstellen, oder
- mindestens ein Indolinderivat der Formel VIb 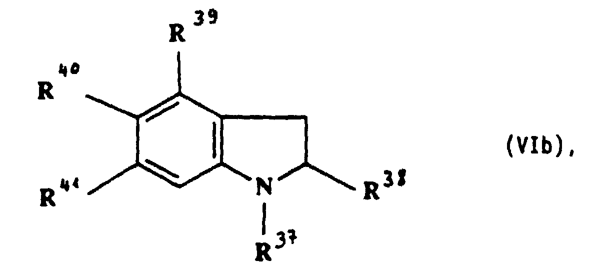 wobei R³⁷ Wasserstoff, eine C₁-C₄-Alkyl- oder C₂-C₄-Acylgruppe, R³⁸ Wasserstoff oder eine Carboxylgruppe bedeutet und R³⁹, R⁴⁰ und R⁴¹ Wasserstoffe, C₁-C₄-Alkylgruppen, C₂-C₄-Acyloxygruppen, Hydroxygruppen, Aminogruppen oder C₁-C₄-Alkoxygruppen darstellen.

12. Mittel nach Anspruch 11, dadurch gekennzeichnet, daß das Indolderivat der Formel VIa bzw. das Indolinderivat der Formel VIb ausgewählt ist aus der Gruppe 5,6-Dihydroxyindol, 5,6-Diacetoxyindol, 4-Hydroxyindolin, 6-Aminoindolin und 5,6-Dihydroxyindolin.

13. Haarfärbemittel in Form eines Pulvers enthaltend mindestens ein Isatinderivat der Formel I in einer Menge von 1 bis 90 Gew.-%, vorzugsweise 20 bis 50 Gew.-%, und eine Aminosäure oder ein aus 2 bis 9 Aminosäuren aufgebautes Oligopeptid in einer Menge von 1 bis 90 Gew.-%, vorzugsweise 20 bis 50 Gew.-%, jeweils bezogen auf das gesamte Pulverfärbemittel.

14. Haarfärbemittel nach Anspruch 13, dadurch gekennzeichnet, daß das Isatinderivat der Formel I ausgewählt ist aus N-Allylisatin, N-Hydroxyisatin und N-(2-Sulfoethyl )-isatin und die Aminosäure bzw. das Oligopeptid ausgewählt sind aus Arginin, Cystein, Methionin, Prolin, Tyrosin, Valin, Glycin, Glutaminsäure, Histidin, Asparaginsäure, Alanin, Tryptophan, Cystin, Lysin, Hydroxyprolin, Leucin, Isoleucin, Phenylalanin, 3,4-Dihydroxyphenylalanin, Serin, Ornithin, Threonin, Glutathion.

15. Haarfärbemittel in Form eines Pulvers enthaltend mindestens ein Isatinderivat der Formel I in einer Menge von 1 bis 90 Gew.-%, vorzugsweise 20 bis 50 Gew.-%, und mindestens ein aromatisches Amin der Formel III in einer Menge von 1 bis 90 Gew.-%, vorzugsweise 20 bis 50 Gew.-%, jeweils bezogen auf das gesamte Pulverfärbemittel.

16. Haarfärbemittel nach Anspruch 15, dadurch gekennzeichnet, daß das Isatinderivat der Formel I ausgewählt ist aus N-Allylisatin, N-Hydroxyisatin und N-(2-Sulfoethyl)-isatin und das aromatische Amin der Formel III ausgewählt ist aus p-Toluylendiamin, 4-Aminophenol, 4-Amino-2-aminomethylphenol, 3,4-Diaminobenzoesäure, 1-(β-Hydroxyethyl)-2,5-diaminobezol.

17. Haarfärbemittel in Form eines Pulvers enthaltend mindestens ein Isatinderivat der Formel I in einer Menge von 1 bis 90 Gew.-%, vorzugsweise 20 bis 50 Gew.-%, und mindestens ein aromatisches Amin der Formel IV in einer Menge von 1 bis 90 Gew.-%, vorzugsweise 20 bis 50 Gew.-%, jeweils bezogen auf das gesamte Pulverfärbemittel.

18. Haarfärbemittel nach Anspruch 17, dadurch gekennzeichnet, daß das Isatinderivat der Formel I ausgewählt ist aus N-Allylisatin, N-Hyroxyisatin, N-(2-Sulfoethyl)-isatin und N-(2-Sulfopropyl)-isatin und das aromatische Amin der Formel IV 1,3-Bis-(2,4-diaminophenoxy)-propan ist.

19. Haarfärbemittel in Form eines Pulvers enthaltend mindestens ein Isatinderivat der Formel I in einer Menge von 1 bis 90, vorzugsweise 20 bis 50 Gew.-%, und mindestens ein Aminopyrimidin der Formel V in einer Menge von 1 bis 90 Gew.-%, vorzugsweise 20 bis 50 Gew.-%, jeweils bezogen auf das gesamte Pulverfärbemittel.

20. Haarfärbemittel nach Anspruch 19, dadurch gekennzeichnet, daß das Isatinderivat der Formel I ausgewählt ist aus N-Allylisatin, N-Hydroxyisatin, N-(2-Sulfoethyl)-isatin und N-(2-Sulfopropyl)-isatin und das Aminopyrimidin der Formel V unsubstituiertes Tetraaminopyrimidin ist.

21. Haarfärbemittel in Form eines Pulvers enthaltend mindestens ein Isatinderivat der Formel I in einer Menge von 1 bis 90 Gew.-%, vorzugsweise 20 bis 50 Gew.-%, und mindestens ein Indolderivat der Formel VIa oder ein Indolinderivat der Formel VIb in einer Menge von 1 bis 90 Gew.-%, vorzugsweise 20 bis 50 Gew.-%, jeweils bezogen auf das gesamte Pulverfärbemittel.

22. Haarfärbemittel nach Anspruch 21, dadurch gekennzeichnet, daß das Isatinderivat der Formel I ausgewählt ist aus N-Allylisatin, N-Hydroxyisatin, N-(2-Sulfoethyl)-isatin und N-(2-Sulfopropyl)-isatin und das Indolderivat der Formel VIa bzw. das Indolinderivat der Formel VIb ausgewählt sind aus 5,6-Dihydroxyindol, 5,6-Diacetoxyindol, 4-Hydroxyindolin, 6-Aminolndolin und 5,6-Dihydroxyindolin.

## Claims

1. The use of isatin derivatives corresponding to formula I: 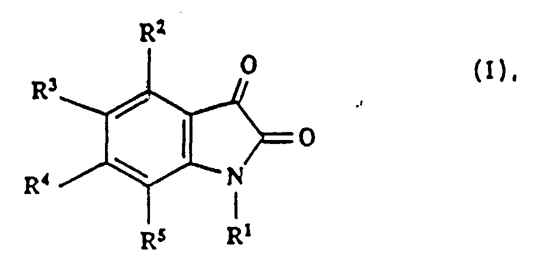 in which R¹ is a hydroxy group, an optionally C₁₋₄₋alkyl- or phenyl-substituted amino group, a C₃₋₈ alkenyl, dihydroxy-(C₃₋₆)-alkyl, trihydroxy-(C₄₋₆)-alkyl, tetrahydroxy-(C₅₋₆)-alkyl, pentahydroxy-C₆-alkyl, C₂₋₄-aminoalkyl, C₁₋₄-sulfoalkyl group, an optionally C₁₋₄-alkyl-substituted 2-furylmethyl, 2-thienylmethyl, 2-pyridylmethyl, 3-pyridylmethyl, 4-pyridylmethyl group or an aralkyl group corresponding to formula (II): 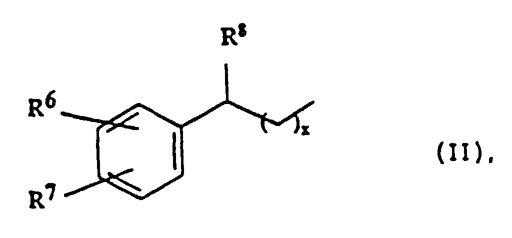 in which R⁶ and R⁷ independently of one another represent hydrogens, halogen atoms, hydroxy groups, amino groups optionally substituted by C₁₋₄ alkyl or phenyl, C₁₋₄ alkyl groups, C₁₋₄ alkoxy groups, carboxy groups or sulfo groups and R⁸ is hydrogen, a C₁₋₄ alkyl group or a C₂₋₄ hydroxyalkyl group and x = 0, 1 or 2,
and R², R³, R⁴ and R⁵ independently of one another represent hydrogens, hydroxy groups, halogen atoms, nitro groups, sulfo groups, carboxyl groups, C₁₋₄ alkyl groups, C₁₋₄ alkoxy groups or NR⁹R¹⁰ groups, where R⁹ and R¹⁰ independently of one another represent hydrogen, C₁₋₄ alkyl groups or C₂₋₄ hydroxyalkyl groups, and two adjacent groups R³, R⁴ and R⁵ may even represent an alkylenedioxy group containing 1 to 4 carbon atoms, and water-soluble salts thereof for coloring keratin-containing fibers.

2. The use of isatin derivatives corresponding to formula I as claimed in claim 1, characterized in that R¹ is an allyl group, a hydroxy group or a 2-sulfoethyl or 2-sulfopropyl group and R² to R⁵ are hydrogens.

3. Formulations for coloring keratin-containing fibers containing at least one isatin derivative corresponding to formula I and at least one amino acid or an oligopeptide made up of 2 to 9 amino acids.

4. A formulation as claimed in claim 3, characterized in that the amino acid or the oligopeptide is selected from the group consisting of arginine, cysteine, methionine, proline, tyrosine, valine, glycine, glutamic acid, histidine, aspartic acid, alanine, tryptophan, cystine, lysine, hydroxyproline, leucine, isoleucine, phenyl alanine, 3,4-dihydroxyphenyl alanine, serine, ornithine, threonine and glutathione.

5. Formulations for coloring keratin-containing fibers containing at least one isatin derivative corresponding to formula I and at least one aromatic amine corresponding to formula III: 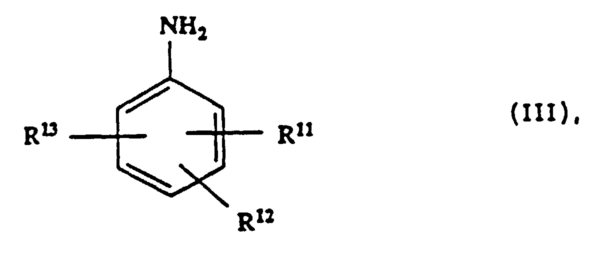 in which R¹¹, R¹² and R¹³ are hydrogens, C₁₋₄ alkyl groups, C₁₋₄ alkoxy groups, hydroxy groups, C₂₋₄ hydroxyalkyl groups, carboxyl groups, sulfo groups, C₁₋₄ aminoalkyl groups or NR¹⁴R¹⁵ groups, where R¹⁴ and R¹⁵ independently of one another are hydrogens, C₁₋₄ alkyl groups, aryl groups or C₂₋₄ hydroxyalkyl groups; two of the groups R¹¹, R¹² and R¹³ together may also form a fused benzene ring optionally substituted by C₁₋₄ alkyl, hydroxy, carboxyl, sulfo, C₁₋₄ aminoalkyl or amino.

6. Formulations as claimed in claim 5, characterized in that the aromatic amine corresponding to formula III is selected from the group consisting of p-tolylenediamine, 4-aminophenol, 4-amino-2-aminomethylphenol, 3,4-diaminobenzoic acid, 1-(β-hydroxyethyl)-2,5-diaminobenzene.

7. Formulations for coloring keratin-containing fibers containing at least one isatin derivative corresponding to formula I and at least one aromatic amine corresponding to formula IV: 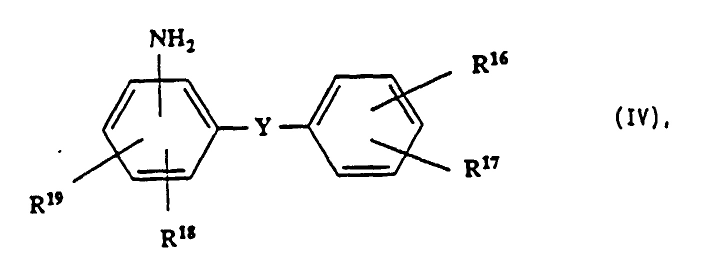 in which Y is a direct bond or a group CO. SO, O, S, NR²⁰, where R²⁰ is hydrogen, C₁₋₄ alkyl or C₂₋₄ hydroxyalkyl, or O-(CH₂-Z-CH₂-O)ₘ, where Z is a direct bond, a group CH₂, CHOH or CH₂OC₂H₄OCH₂ and m is an integer of 1 to 4, or Y may even be a saturated or unsaturated alkylene group containing 1 to 4 carbon atoms which may optionally be substituted by OH and R¹⁶, R¹⁷, R¹⁸ and R¹⁹ are hydrogens, C₁₋₄ alkyl groups, C₂₋₄ hydroxyalkyl groups, C₂₋₄-(C₁₋₄-alkoxy)-alkyl groups or groups NR²¹R²² or OR²³, where R²¹, R²² and R²³ independently of one another represent hydrogens, C₁₋₄ alkyl groups, C₁₋₄ alkoxy groups, C₂₋₄ hydroxyalkyl groups or C₂₋₄-(C₁₋₄-alkoxy)-alkyl groups, with the proviso that at least one of the groups R¹⁶ and R¹⁷ and one of the groups R¹⁸ and R¹⁹ is a group NR²¹R²² or OR²³.

8. Formulations as claimed in claim 7, characterized in that the aromatic amine corresponding to formula IV is 1,3-bis-(2,4-diaminophenoxy)-propane, 1,8-bis-(2,5-diaminophenoxy)-3,6-dioxaoctane or 4,4'-diaminodiphenytamine.

9. Formulations for coloring keratin-containing fibers containing at least one isatin derivative corresponding to formula I and at least one aminopyrimidine corresponding to formula V: 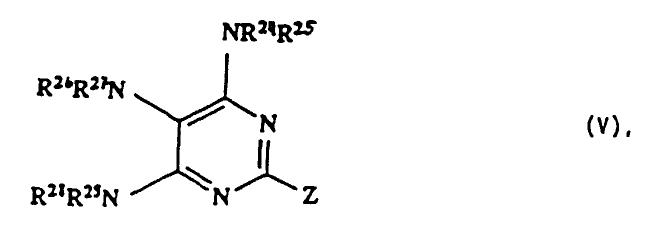 in which R²⁴, R²⁵, R²⁶, R²⁷, R²⁸ and R²⁹ independently of one another are hydrogens, C₁₋₄ alkyl groups or C₂₋₄ hydroxyalkyl groups and Z is hydrogen, an OH group or an NR³⁰R³¹ group, where R³⁰ and R³¹ independently of one another are hydrogens, C₁₋₄ alkyl groups or C₂₋₄ hydroxyalkyl groups.

10. Formulations as claimed in claim 9, characterized in that the substituents R²⁴ to R²⁹ in the tetraaminopyrimidine corresponding to formula V are hydrogens.

11. Formulations for coloring keratin-containing fibers containing at least one isatin derivative corresponding to formula I and
- at least one indole derivative corresponding to formula VIa: 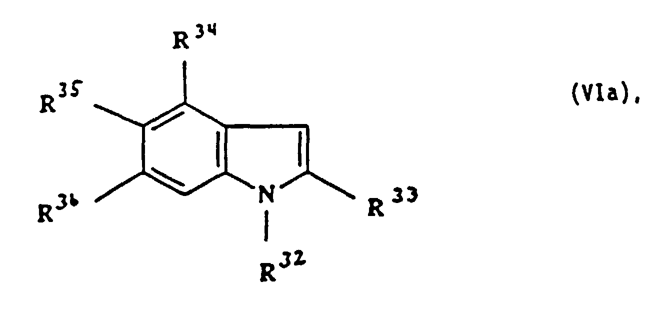 in which R³² is hydrogen, a C₁₋₄ alkyl or C₂₋₄ acyl group, R³³ is hydrogen or a carboxyl group and R³⁴, R³⁵ and R³⁶ are hydrogens, C₁₋₄ alkyl groups, C₂₋₄ acyloxy groups, hydroxy groups, amino groups or C₁₋₄ alkoxy groups, or
- at least one indoline derivative corresponding to formula VIb: 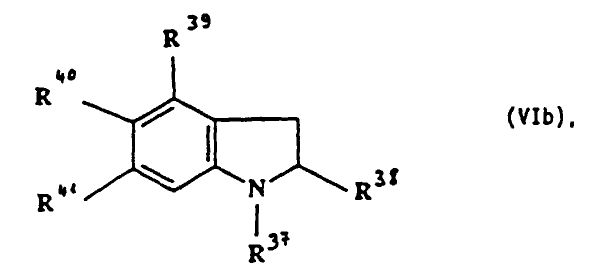 in which R³⁷ is hydrogen, a C₁₋₄ alkyl or C₂₋₄ acyl group, R³⁸ is hydrogen or a carboxyl group and R³⁹, R⁴⁰ and R⁴¹ are hydrogens, C₁₋₄ alkyl groups, C₂₋₄ acyloxy groups, hydroxy groups, amino groups or C₁₋₄ alkoxy groups.

12. Formulations as claimed in claim 11, characterized in that the indole derivative corresponding to formula VIa or the indoline derivative corresponding to formula VIb is selected from the group consisting of 5,6-dihydroxyindole, 5,6-diacetoxyindole, 4-hydroxyindoline, 6-aminoindoline and 5,6-dihydroxyindoline.

13. Hair coloring formulations in the form of a powder containing at least one isatin derivative corresponding to formula I in a quantity of 1 to 90% by weight and preferably 20 to 50% by weight and an amino acid or an oligopeptide made up of 2 to 9 amino acids in a quantity of 1 to 90% by weight and preferably 20 to 50% by weight, based on the powder-form coloring formulation as a whole.

14. Hair coloring formulations as claimed in claim 13, characterized in that the isatin derivative corresponding to formula I is selected from N-allyl isatin, N-hydroxyisatin and N-(2-sulfoethyl)-isatin and the amino acid or the oligopeptide is selected from arginine, cysteine, methionine, proline, tyrosine, valine, glycine, glutamic acid, histidine, aspartic acid, alanine, tryptophan, cystine, lysine, hydroxyproline, leucine, isoleucine, phenyl alanine, 3,4-dihydroxyphenyl alanine, serine, omithine, threonine and glutathione.

15. Hair coloring formulations in the form of a powder containing at least one isatin derivative corresponding to formula I in a quantity of 1 to 90% by weight and preferably 20 to 50% by weight and at least one aromatic amine corresponding to formula III in a quantity of 1 to 90% by weight and preferably 20 to 50% by weight, based on the powder-form coloring formulation as a whole.

16. Hair coloring formulations as claimed in claim 15, characterized in that the isatin derivative corresponding to formula I is selected from N-allyl isatin, N-hydroxyisatin and N-(2-sulfoethyl)-isatin and the aromatic amine corresponding to formula III is selected from the group consisting of p-tolylenediamine, 4-aminophenol, 4-amino-2-aminomethylphenol, 3,4-diaminobenzoic acid, 1-(β-hydroxyethyl)-2,5-diaminobenzene.

17. Hair coloring formulations in the form of a powder containing at least one isatin derivative corresponding to formula I in a quantity of 1 to 90% by weight and preferably 20 to 50% by weight and at least one aromatic amine corresponding to formula IV in a quantity of 1 to 90% by weight and preferably 20 to 50% by weight, based on the powder-form coloring formulation as a whole.

18. Hair coloring formulations as claimed in claim 17, characterized in that the isatin derivative corresponding to formula I is selected from N-allyl isatin, N-hydroxyisatin, N-(2-sulfoethyl)-isatin and N-(2-sulfopropyl)-isatin and the aromatic amine corresponding to formula IV is 1,3-bis-(2,4-diaminophenoxy)-propane.

19. Hair coloring formulations in the form of a powder containing at least one isatin derivative corresponding to formula I in a quantity of 1 to 90% by weight and preferably 20 to 50% by weight and at least one aminopyrimidine corresponding to formula V in a quantity of 1 to 90% by weight and preferably 20 to 50% by weight, based on the powder-form coloring formulation as a whole.

20. Hair coloring formulations as claimed in claim 19, characterized in that the isatin derivative corresponding to formula I is selected from N-allyl isatin, N-hydroxyisatin, N-(2-sulfoethyl)-isatin and N-(2-sulfopropyl)-isatin and the aminopyrimidine corresponding to formula V is unsubstituted tetraaminopyrimidine.

21. Hair coloring formulations in the form of a powder containing at least one isatin derivative corresponding to formula I in a quantity of 1 to 90% by weight and preferably 20 to 50% by weight and at least one indole derivative corresponding to formula VIa or an indoline derivative corresponding to formula VIb in a quantity of 1 to 90% by weight and preferably 20 to 50% by weight, based on the powder-form coloring formulation as a whole.

22. Hair coloring formulations as claimed in claim 21, characterized in that the isatin derivative corresponding to formula I is selected from N-allyl isatin, N-hydroxyisatin, N-(2-sulfoethyl)-isatin and N-(2-sulfopropyl)-isatin and the indole derivative corresponding to formula VIa or the indoline derivative corresponding to formula Vib is selected from the group consisting of 5,6-dihydroxyindole, 5,6-diacetoxyindole, 4-hydroxyindoline, 6 aminoindoline and 5,6-dihydroxyindoline.

## Revendications

1. Utilisation de dérivés d'isatine de formule I : 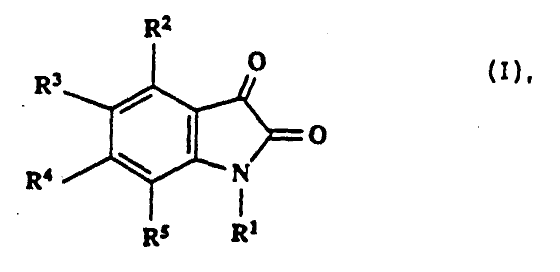 dans laquelle R¹ représente un groupe hydroxy, un groupe amino éventuellement substitué par un alkyle en C₁ à C₄ ou un phényle, un groupe alcényle en C₃ à C₈, dihydroxy-alkyle en C₃ à C₆, trihydroxy-alkyle en C₄ à C₆, tétrahydroxyalkyle en C₅ à C₆, pentahydroxyalkyle en C₆, aminoalkyle en C₂ à C₄, sulfoalkyle en C₁ à C₄, un radical 2-furylméthyle, 2-thiénylméthyle, 2-pyridylméthyle, 3-pyridylméthyle, ou 4-pyridylméthyle éventuellement substitué par un alkyle en C₁ à C₄ ou un groupe aralkyle de formule (II) : 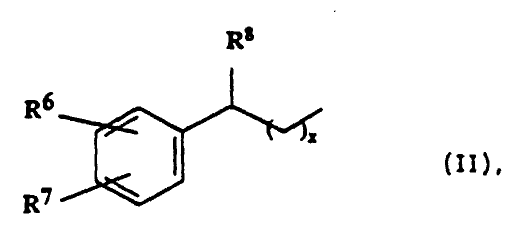 dans laquelle R⁶ et R⁷ représentent indépendamment l'un de l'autre des atomes d'hydrogène, des atomes d'halogène, des groupes hydroxy, des groupes amino, qui le cas échéant peuvent être substitués par un alkyle en C₁ à C₄ ou un phényle, des groupes alkyle en C₁ à C₄, des groupes alcoxy en C₁ à C₄, des groupes carboxy ou des groupes sulfo et R⁸ représente un hydrogène, un groupe alkyle en C₁ à C₄ ou un groupe hydroxyalkyle en C₂ à C₄ et x vaut 0, 1 ou 2, et R², R³, R⁴ et R⁵ représentent indépendamment l'un de l'autre des atomes d'hydrogène, des groupes hydroxy, des atomes d'halogène, des groupes nitro, des groupes sulfo, des groupes carboxyle, des groupes alkyle en C₁ à C₄, des groupes alcoxy en C₁ à C₄ ou des groupes NR⁹R¹⁰, ou R⁹ et R¹⁰ représentent indépendamment l'un de l'autre un hydrogène, des groupes alkyle en C₁ à C₄ ou des groupes hydroxyalkyle en C₂ à C₄, et deux groupes R³, R⁴ et R⁵ voisins peuvent également représenter un groupe alkylène dioxy ayant de 1 à 4 atomes de carbone, et de leurs sels solubles dans l'eau pour la coloration des fibres contenant de la kératine.

2. Utilisation de dérivés d'isatine de formule I selon la revendication 1,
caractérisée en ce que
R¹ représente un groupe allyle, un groupe hydroxy ou un groupe 2-sulfoéthyle ou -propyle et R² à R⁵ représentent des atomes d'hydrogène.

3. Agent de coloration de fibres contenant de la kératine, qui contient au moins un dérivé d'isatine de formule I et au moins un acide aminé ou un oligopeptide constitué de 2 à 9 acides aminés.

4. Agent selon la revendication 3,
caractérisé en ce que
l'acide aminé ou l'oligopeptide sont choisis dans le groupe constitué par l'arginine, la cystéine, la méthionine, la proline, la tyrosine, la valine, la glycine, l'acide glutamique, l'histidine, l'acide aspartique, l'alanine, le tryptophane, la cystine, la lysine, l'hydroxyproline, la leucine, l'isoleucine, la phénylalanine, la 3,4-dihydroxyphénylalanine, la sérine, l'ornithine, la thréonine, le glutathion.

5. Agent de coloration des fibres contenant de la kératine, qui contient au moins un dérivé d'isatine de formule I et au moins une amine aromatique de formule III : 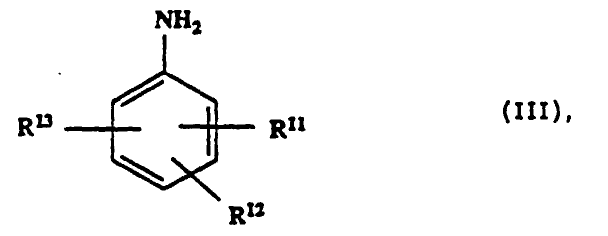 dans laquelle R¹¹, R¹² et R¹³ représentent des atomes d'hydrogène, des groupes alkyle en C₁ à C₄, des groupes alcoxy en C₁ à C₄, des groupes hydroxy, des groupes hydroxyalkyle en C₂ à C₄, des groupes carboxyle, des groupes sulfo, des groupes aminoalkyle en C₁ à C₄ ou des groupes NR¹⁴ R¹⁵, où R¹⁴ et R¹⁵ représentent indépendamment l'un de l'autre des atomes d'hydrogène, des groupes alkyle en C₁ à C₄, des groupes aryle, ou des groupes hydroxyalkyle en C₂ à C₄, où deux des groupes R¹¹, R¹² et R¹³ peuvent également former ensemble un noyau benzénique condensé qui est éventuellement substitué par un alkyle en C₁ à C₄, un hydroxy, un carboxy, un sulfo, un aminoalkyle en C₁ à C₄ ou un amino.

6. Agent selon la revendication 5,
caractérisé en ce que
l'amine aromatique de formule III est choisie dans le groupe constitué par la p-toluylène diamine, le 4-aminophénol, le 4-amino-2-aminométhylphénol, l'acide 3,4-diaminobenzoïque, le 1-(β-hydroxyéthyl)-2,5-diaminobenzène.

7. Agent de coloration des fibres contenant de la kératine qui contient au moins un dérivé d'isatine de formule I et au moins une amine aromatique de formule IV : 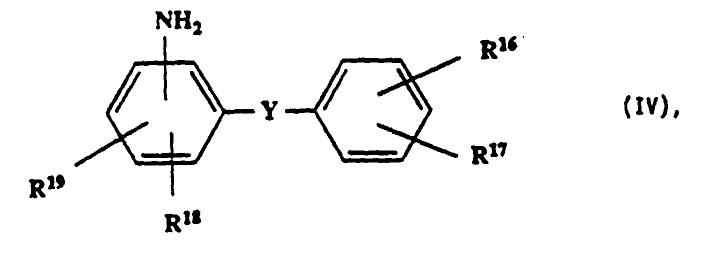 dans laquelle Y représente une liaison directe ou un groupe CO, SO, O, S, NR²⁰ dans lequel R²⁰ représente un atome d'hydrogène, un alkyle en C₁ à C₄ ou un hydroxy alkyle en C₂ à C₄, ou O-(CH₂-Z-CH₂-O)ₘ, où Z représente une liaison directe, un groupe CH₂, CHOH ou CH₂OC₂H₄OCH₂, et m représente un nombre entier allant de 1 à 4, ou bien Y représente encore un groupe alkylène saturé ou insaturé ayant de 1 à 4 atomes de carbone, qui peut le cas échéant être substitué par OH, et R¹⁶, R¹⁷, R¹⁸ et R¹⁹ représentent des atomes d'hydrogène, des groupes alkyle en C₁ à C₄, des groupes hydroxyalkyle en C₂ à C₄, des groupes (alcoxy en C₁ à C₄)-alkyle en C₂ à C₄ ou des groupes NR²¹ R²² ou OR²³, où R²¹, R²² et R²³ représentent indépendamment l'un de l'autre des atomes d'hydrogène, des groupes alkyle en C₁ à C₄, des groupes alcoxy en C₁ à C₄, des groupes hydroxyalkyle en C₂ à C₄ ou des groupes (alcoxy en C₁ à C₄)-alkyle en C₂ à C₄, sous réserve qu'au moins un des groupes R¹⁶ et R¹⁷ et un des groupes R¹⁸ et R¹⁹ représente à chaque fois un groupe NR²¹R²² ou OR²³.

8. Agent selon la revendication 7,
caractérisé en ce que
l'amine aromatique de formule IV est le 1,3-bis-(2,4-diaminophénoxy)propane, le 1,8-bis-(2,5-diaminophénoxy)-3,6-dioxaoctane ou la 4,4'-diaminodiphénylamine.

9. Agent de coloration des fibres contenant de la kératine qui contient au moins un dérivé d'isatine de formule I et au moins une aminopyrimidine de formule V : 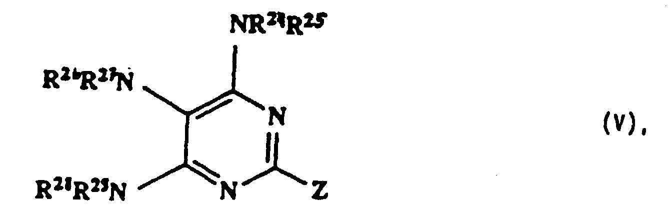 dans laquelle R²⁴, R²⁵, R²⁶, R²⁷, R²⁸ et R²⁹ représentent indépendamment l'un de l'autre des atomes d'hydrogène, des groupes alkyle en C₁ à C₄ ou des groupes hydroxyalkyle en C₂ à C₄ et Z représente un atome d'hydrogène, un groupe OH ou un groupe NR³⁰ R³¹, où R³⁰ et R³¹ représentent indépendamment l'un de l'autre des atomes d'hydrogène, des groupes alkyle en C₁ à C₄ et des groupes hydroxyalkyle en C₂ à C₄.

10. Agent selon la revendication 9,
caractérisé en ce que
dans la tétraaminopyrimidine de formule V les groupes R²⁴ à R²⁹ représentent des atomes d'hydrogène.

11. Agent de coloration de fibres contenant de la kératine contenant au moins un dérivé d'isatine de formule I et au moins un dérivé d'indole de formule VIa : 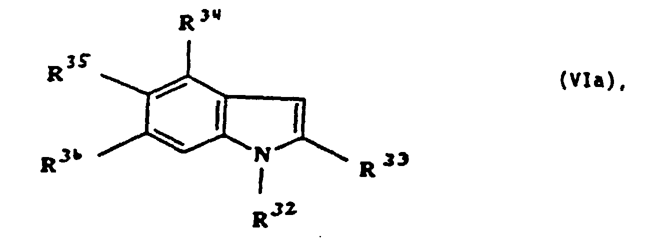 dans laquelle R³² représente un hydrogène, un groupe alkyle en C₁ à C₄ ou un groupe acyle en C₂ à C₄, R³³ représente un atome d'hydrogène ou un groupe carboxyle et R³⁴, R³⁵ et R³⁶ représentent des atomes d'hydrogène, des groupes alkyle en C₁ à C₄, des groupes acyloxy en C₂ à C₄, des groupes hydroxy, des groupes amino ou des groupes alcoxy en C₁ à C₄, ou au moins un dérivé d'indoline de formule VIb : 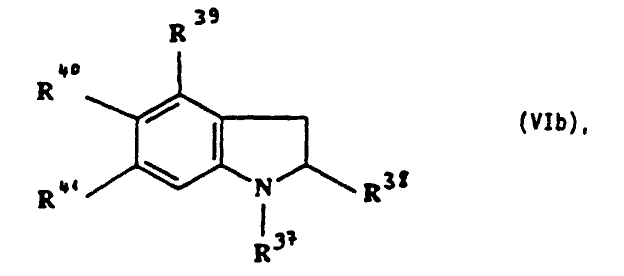 dans laquelle R³⁷ représente un hydrogène, un groupe alkyle en C₁ à C₄ ou acyle en C₂ à C₄, R³⁸ représente un hydrogène ou un groupe carboxyle et R³⁹, R⁴⁰ et R⁴¹ représentent des atomes d'hydrogène, des groupes alkyle en C₁ à C₄, des groupes acyloxy en C₂ à C₄, des groupes hydroxy, des groupes amino ou des groupes alcoxy en C₁ à C₄.

12. Agent selon la revendication 11,
caractérisé en de que
le dérivé d'indole de formule VIa ou selon les cas le dérivé d'indoline de formule VIb est choisi dans le groupe du 5,6-dihydroxyindole, du 5,6-diacétoxyindole, de la 4-hydroxyindoline, de la 6-aminoindoline et de la 5,6-dihydroxyindoline.

13. Agent de coloration des cheveux sous forme de poudre contenant au moins un dérivé d'isatine de formule I en une quantité de 1 à 90 % en poids et de préférence de 20 à 50 % en poids, et un acide aminé ou un oligopeptide constitué de 2 à 9 acides aminés en une quantité de 1 à 90 % en poids, de préférence de 20 à 50 % en poids, toujours par rapport à l'ensemble du colorant pulvérulent.

14. Agent de coloration des cheveux selon la revendication 13,
caractérisé en ce que
le dérivé d'isatine de formule I est choisi parmi la L-allylisatine, la N-hydroxyisatine et la N-(2-sulfoéthyl)-isatine et l'acide aminé ou selon les cas l'oligopeptide est choisi parmi l'arginine, la cystéine, la méthionine, la proline, la tyrosine, la valine, la glycine, l'acide glutamique, l'histidine, l'acide aspartique, l'alanine, la tryptophane, la cystine, la lysine, l'hydroxyproline, la leucine, l'isoleucine, la phénylalanine, la 3,4-dihydroxyphénylalanine, la sérine, l'ornithine, la thréonine, le glutathion.

15. Agent de coloration des cheveux sous la forme d'une poudre contenant au moins un dérivé d'isatine de formule I en une quantité de 1 à 90 % en poids, de préférence de 20 à 50 % en poids, et au moins une amine aromatique de formule III en une quantité de 1 à 90 % en poids, de préférence de 20 à 50 % en poids, toujours par rapport à l'ensemble du colorant pulvérulent.

16. Agent de coloration des cheveux selon la revendication 15,
caractérisé en ce que
le dérivé d'isatine de formule I est choisi parmi la N-allylisatine, la N-hydroxyisatine et la N-(2-sulfoéthyl)-isatine et en ce que l'amine aromatique de formule III est choisie parmi la p-toluylènediamine, le 4-aminophénol, le 4-amino-2-aminométhylphénol, l'acide 3,4-diaminobenzoïque, le 1-(β-hydroxyéthyl)-2,5-diaminobenzène.

17. Agent de coloration des cheveux sous la forme d'une poudre contenant au moins un dérivé d'isatine de formule I en une quantité de 1 à 90% en poids, de préférence de 20 à 50 % en poids, et au moins une amine aromatique de formule IV en une quantité de 1 à 90 % en poids, de préférence de 20 à 50 % en poids, toujours par rapport à l'ensemble du colorant pulvérulent.

18. Agent de coloration des cheveux selon la revendication 17,
caractérisé en ce que
le dérivé d'isatine de formule I est choisi parmi la N-allylisatine, la N-hydroxyisatine, la N-(2-sulfoéthyl)-isatine et la N-(2-sulfopropyl)-isatine et en ce que l'amine aromatique de formule IV est le 1,3-bis-(2,4-diaminophénoxy)-propane.

19. Agent de coloration des cheveux sous la forme d'une poudre contenant au moins un dérivé d'isatine de formule I en une quantité de 1 à 90, de préférence de 20 à 50 % en poids, et au moins une aminopyrimidine de formule V, en une quantité de 1 à 90 % en poids, de préférence de 20 à 50 % en poids, toujours par rapport à l'ensemble du colorant pulvérulent.

20. Colorant pour cheveux selon la revendication 19,
caractérisé en ce que
le dérivé d'isatine de formule I est choisi parmi la N-allylisatine, la N-hydroxyisatine, la N-(2-sulfoéthyl)-isatine et la N-(2-sulfopropyl)-isatine, et l'aminopyrimidine de formule V est la tétraaminopyrimidine non substituée.

21. Agent de coloration des cheveux sous la forme d'une poudre contenant au moins un dérivé d'isatine de formule I en une quantité de 1 à 90 % en poids, de préférence de 20 à 50 % en poids, et au moins un dérivé d'indole de formule VIa ou un dérivé d'indoline de formule VIb en une quantité de 1 à 90 % en poids, de préférence de 20 à 50 % en poids, toujours par rapport à l'ensemble du colorant pulvérulent.

22. Colorant pour cheveux selon la revendication 21,
caractérisé en ce que
le dérivé d'isatine de formule I est choisi parmi la N-allylisatine, la N-hydroxyisatine, la N-(2-sulfoéthyl)-isatine et la N-(2-sulfopropyl)-isatine et en ce que le dérivé d'indole de formule VIa ou le dérivé d'indoline de formule VIb est choisi parmi le 5,6-dihydroxyindole, le 5,6-diacétoxyindole, la 4-hydroxyindoline, la 6-aminoindoline et la 5,6-dihydroxyindoline.
